# EUROPEAN PATENT APPLICATION

(11) **EP 2 305 343 A2**
(43) Date of publication of application: **06.04.2011**
(21) Application number: 09731759.8
(22) Date of filing: 17.04.2009
(51) Int. Cl.: A61M 39/22, A61M 1/00, A61M 27/00, A61M 16/04

(54) **DEVICE FOR CONTROLLING AND MONITORING VACUUM PRESSURE IN SYSTEMS FOR THE SUCTION OF BIOLOGICAL SECRETIONS**

(30) Priority: 17.04.2008 BR PI0802006
(71) Applicant: Universidade Federal de Minas Gerais-Ufmg, Campus UFMG - Pampulha 31270-901 Belo Horizonte, MG (BR)
(72) Inventor: PINOTTI BARBOSA, Marcos, Belo Horizonte - MG (BR); LIMA CAMPOS, Shirley, Belo Horizonte - MG (BR); CARVALHO SOARES, Fabrício, Belo Horizonte - MG (BR); BARBOSA DE DEUS, José Renato, Belo Horizonte - MG (BR)
(74) Representative: Winter, Brandl, Fürniss, Hübner, Röss, Kaiser, Polte - Partnerschaft
(86) International application number: PCT/BR2009/000111
(87) International publication number: WO 2009/127028

(57) **Abstract**

The present invention concerns a vacuum pressure control and monitoring device for aspiration of biological secretions from respiratory routes, as well as of other biological fluids. The proposed device consists of a vacuum pressure regulating valve integrated to a monitoring system, which can be coupled together a hospital vacuum network and portable aspiration apparatus in ambulatory and household environments. The said device is designed to control and monitor maximum vacuum aspiration pressures, in this way preventing injuries during aspiration procedures.

## Description

The present invention concerns a vacuum pressure control and monitoring device for aspiration of biological secretions from respiratory routes, as well as of other biological fluids. The proposed device consists of a vacuum pressure regulating valve integrated to a monitoring system, which can be coupled together a hospital vacuum network and portable aspiration apparatus in ambulatory and household environments. The said device is designed to control and monitor maximum vacuum aspiration pressures, in this way preventing injuries during aspiration procedures.

The vacuum pressure hereto refers to the module of absolute pressure (absolute value) of the subtracted absolute pressure of local atmospheric pressure. Therefore, a small value of vacuum pressure means a pressure that is somewhat below the atmospheric pressure. Correspondingly, a higher value of vacuum pressure means a pressure well below the atmospheric pressure.

Aspiration of respiratory routes consists in introducing a catheter connected to a vacuum source into an individual respiratory route in order to remove respiratory tract secretions. The aforesaid aspiration is a simple technical procedure routinely used in patients who are unable to voluntarily expel respiratory tract secretions. However, vacuum pressure must be held within certain limits so as to prevent tissue traumas.

Excessive pressure during respiratory route aspiration may cause the mucous membrane to be sucked into the catheter holes in this way injuring the tracheal wall.

Vacuum pressures ranging from 100 to 120 mmHg for adults, 80 to 100 mmHg for children and 60 to 80 mmHg for lactant babies are recommended.

Some patents describe the development of aspirators for biological application, such as the exhausted patent MU6902511-8, which concerns an electric equipment designed for aspiration of abdominal and pleural secretions in surgical procedures. As for other vacuum pressure control systems, patent PI8701126 describes a vacuum control device regulated by constant electrical resistance, whereas patent MU7901435-6 refers to a vacuum control device having a constant water volume for aspiration drainage, although both of them are not applicable as far as aspiration procedures for respiration routes are concerned.

Patent PI0303078-4 is concerned with a device designed for suction pressure graduation of microsurgical aspirators, consisting of a fixed tube with orifices, upon which another tube, connected to a blade, slides. The vacuum pressure control occurs as the blade and mobile tube slide on the fixed tube, driven by the surgeon's thumb movements.

Some aspiration systems for respiration routes in hospital environments control vacuum pressure with the use of flow regulators, whose performance is inefficacious however, as this kind of control is unable to limit the maximum vacuum of aspiration due to the system's dynamic behavior.

In the face of aspiration systems which cannot efficiently control vacuum pressure, a device is proposed here that prevents mucosa membrane traumas caused by excessive vacuum pressures during aspiration procedures of biological secretions and fluids. This mechanical device possesses a valve comprised of a piston and springs that can be coupled together several aspiration systems for respiration routes or biological fluids and that allows vacuum pressure being adjusted and held constant during a surgical procedure.

This device can also be adapted to both large systems, such as hospital systems, and portable systems, used in ambulatory or household environments.

The figures listed below illustrate the vacuum pressure control and monitoring device, which are the object of the present invention patent request herein, as follows:

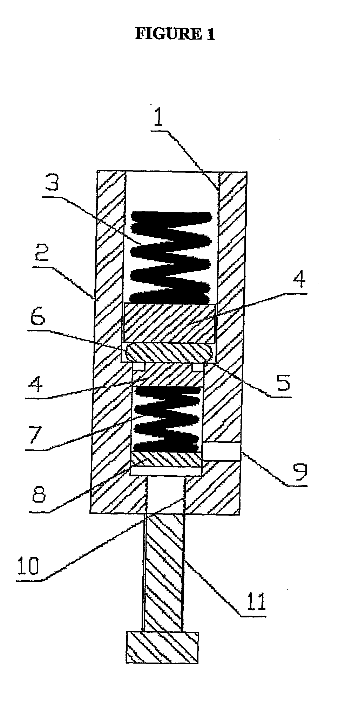

Figure 1 shows the vacuum pressure regulating valve.

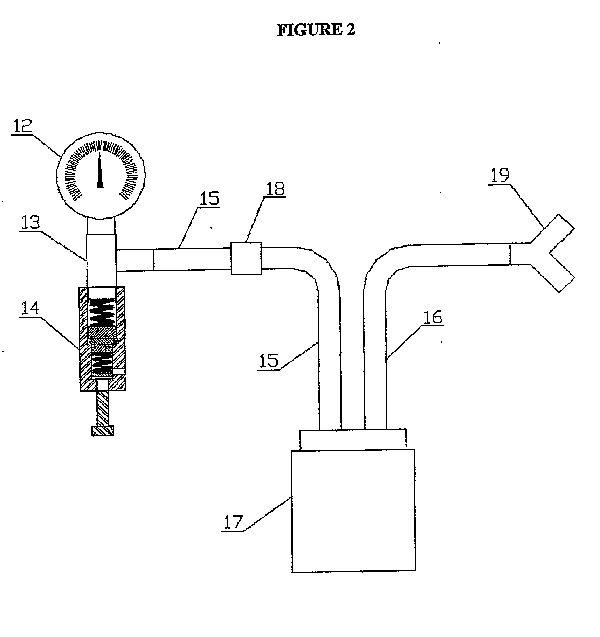

Figure 2 shows the monitoring system integrated to the vacuum pressure regulating valve.

As can be seen in (1), an outer connection thread is designed to coupling the valve together the aspiration systems. The valve body (2) can be dimensioned so as to be coupled together with any kind of outer thread and any pressure or flowing value. In (3) a spring can be seen, whose resulting strength exerted on the piston (4) should be equal to the pressure strength value exerted on this very piston by the opposite side of the spring added up to the highest aspiration pressure value desired. The sealing seat (5) is placed on the valve body (2) by means of any nonrestrictive manufacturing method, such as milling, electro erosion, among others.

A sealing ring is seen in (6), whose function is to avoid leakages while the system is closed. A spring (7) is responsible for regulating the valve, which allows the increasing or decreasing valve vacuum pressure. A bumper is shown in (8), which is designed for transmitting the regulation bolt (11) movement to the spring (7). The air entry orifice (9) can be open to the atmosphere or connected to any other pressure system. The thread seen in (10) is cut on the regulation bolt (11).

When the regulation bolt (11) completely open, the maximum aspiration pressure is equal to the entry pressure in the orifice (9) multiplied by the piston smallest area (4) subtracted by the strength that the spring (3) exerts on such piston. As the bolt (11) is driven clockwise, the spring (7) is compressed and more strength is exerted onto the piston (4). Such strength lessens vacuum pressure, which makes it possible that the same aspiration pressure control valve can be used in different situations, such as secretion aspiration in adults, children and newborns.

Once the adjustment and gauging of the springs of the vacuum pressure regulating valve are carried out, the vacuum pressure, which is generated during aspiration of respiration routes, can be assessed by the monitoring system integrated to the proposed device.

The monitoring system comprises a vacuum pressure reader (12), which reads and/or records the related data and can be operated by using three different nonrestrictive **methods:** a first one using mechanical principles, with a Bourdon tube vacuumeter; a second using electrical principles with a differential piezoresistive pressure sensor or another electromagnetic effect connected to a data acquisition plate, with the local atmospheric pressure taken as reference pressure; or a third using optical principles as a differential pressure system based on optical fibers or other effect based on disturbance in the propagation of light in a wave fiber or guide, all previously calibrated, taking local atmospheric pressure as the reference pressure.

For its operation, the monitoring system is connected to a liquid separator container (17) with an antimicrobial filter (18) both connected to the vacuum pressure regulating valve by crystal-clear polyvinyl chloride (PVC) hoses and appropriate connections.

As seen in (13), the T connection - which can be dimensioned for any kind of outer thread - allows a simultaneous coupling of the vacuum pressure reader (12), the vacuum pressure regulating valve (14) and the aspiration catheter (15). The vacuum pressure regulating valve (14) has springs (3) and (7) exerting strength on the piston (4), which regulate the vacuum pressure.

The aspiration line (15) and (16), comprised of crystal PVC hoses, or other nonrestrictive transparent material, allows visualizing the aspiration circuit besides serving as connectors between the monitoring system elements. The aspiration line possesses two components: the smallest circuit (15), which is extended from the T connection (13) to the liquid separator reservoir (17); and the largest circuit (16), which is extended from the liquid separator reservoir (17) to the Y adaptor (19).

The liquid separator container (17) is the recipient where the aspiration line circuits (15) and (16) discharge. The said container is necessary in order to prevent that the sudden entry of the atmospheric air push part of the fluid into the system flow toward the vacuum pressure reader, which would compromise its functioning when the system is depressurized at the end of the aspiration procedure. Such a possibility also justifies the use of a smaller circuit (15), which is extended from the liquid separator container (17) to the T connection (13).

An antimicrobial filter (18), localized in the aspiration line circuit (15), is used for preventing that solid or liquid residues reach the pressure sensor/transducer, in this way reducing eventual malfunctioning or contamination by microorganisms.

A Y adaptor (19) is connected to the extremity (16) of the aspiration line circuit, in order to allow the whole system coupling to the vacuum pressure generator (secretion aspirator for hospital or portable use) and to the aspiration catheter introduced into the patients' respiration routes.

In addition to allowing vacuum pressure data assessment in real time all through the secretion aspiration procedure, the monitoring system also enables preventive inspection, maintenance and reassessment of the functioning of the vacuum pressure regulating valve.

Furthermore, the monitoring system can perform the following actions in real time: to identify minimum, medium and maximum vacuum pressures; detect and quantify pressure peaks; observe the vacuum pressure dynamic behavior during aspiration; readjust the vacuum pressure under the valve control, when necessary; quantify eventual charge loss in the microbial filter; and, in this way, it can indicate the need to replace device components.

## Claims

1. **"VALVE TO CONTROL THE NEGATIVE PRESSURE OF ASPIRATION SYSTEM OF BIOLOGICAL SECRETIONS",** comprising a valve (2), which allows vacuum pressure regulation in an aspiration system within the measuring range of 60 to 120 mmHg.

2. **"VALVE TO CONTROL THE NEGATIVE PRESSURE OF ASPIRATION SYSTEM OF BIOLOGICAL SECRETIONS",** according to claim 1, wherein the valve (2) comprise an outer thread (1) cut on the aforesaid valve for its connection to the aspiration systems.

3. **"VALVE TO CONTROL THE NEGATIVE PRESSURE OF ASPIRATION SYSTEM OF BIOLOGICAL SECRETIONS",** according to claim 2, wherein the aforesaid valve body (2) be matched with any kind of outer thread and suitable for any pressure and discharge value.

4. **"VALVE TO CONTROL THE NEGATIVE PRESSURE OF ASPIRATION SYSTEM OF BIOLOGICAL SECRETIONS"**, according to claim 1, comprising a spring (3) that controls the piston(4) positioning.

5. **"VALVE TO CONTROL THE NEGATIVE PRESSURE OF ASPIRATION SYSTEM OF BIOLOGICAL SECRETIONS",** according to claim 1, comprising a sealing seat (5) conformed to the valve body.

6. **"VALVE TO CONTROL THE NEGATIVE PRESSURE OF ASPIRATION SYSTEM OF BIOLOGICAL SECRETIONS",** according to claim 1, comprising a sealing ring (6) to prevent leakages in the system while closed or in operation.

7. **"VALVE TO CONTROL THE NEGATIVE PRESSURE OF ASPIRATION SYSTEM OF BIOLOGICAL SECRETIONS",** according to claim 1, comprising a spring (7) and a regulation bolt (11) that are responsible for the aforesaid vacuum pressure adjustment.

8. **"VALVE TO CONTROL THE NEGATIVE PRESSURE OF ASPIRATION SYSTEM OF BIOLOGICAL SECRETIONS",** according to claim 1, comprising a bumper (8) responsible for transmitting the regulation bolt (11) movement to the spring (7).

9. **"VALVE TO CONTROL THE NEGATIVE PRESSURE OF ASPIRATION SYSTEM OF BIOLOGICAL SECRETIONS",** according to claim 1, wherein the system's vacuum pressure decrease being carried out with a clockwise turn of the bolt (11), in this way compressing the spring (7) and increasing the strength exerted onto the piston (4).

10. **"VALVE TO CONTROL THE NEGATIVE PRESSURE OF ASPIRATION SYSTEM OF BIOLOGICAL SECRETIONS",** according to claim 1, wherein the system's vacuum pressure increase being carried out with an anticlockwise turn of the bolt (11), in this way decompressing the spring (7) and diminishing the strength exerted onto the piston (4).

11. **"VALVE TO CONTROL THE NEGATIVE PRESSURE OF ASPIRATION SYSTEM OF BIOLOGICAL SECRETIONS",** according to claim 1, comprising an air entry orifice (9) that may be opened to the atmosphere or linked to any other pressure system.

12. **"VALVE TO CONTROL THE NEGATIVE PRESSURE OF ASPIRATION SYSTEM OF BIOLOGICAL SECRETIONS",** according to claim 1, which can be applied to the respiratory routes aspiration in adults, children and newborns.

13. **"DEVICE TO CONTROL THE SUCTION PRESSURE IN THE SYSTEM PERFORMING ASPIRATION OF SECRETIONS FROM THE RESPIRATORY TRACT",** comprising the control and monitoring the negative pressure (vacuum) during secretions aspiration procedures of respiratory routes or any other biological fluids by using the valve indicated in claims 1 to 12 integrated to a monitoring system.

14. **"DEVICE TO CONTROL THE SUCTION PRESSURE IN THE SYSTEM PERFORMING ASPIRATION OF SECRETIONS FROM THE RESPIRATORY TRACT",** according to claim 13, comprising a vacuum pressure measuring system (12), responsible for data acquisition and recording, which can consist of a mechanical, electromagnetic or optical system previously calibrated.

15. **"DEVICE TO CONTROL THE SUCTION PRESSURE IN THE SYSTEM PERFORMING ASPIRATION OF SECRETIONS FROM THE RESPIRATORY TRACT",** according to claim 13, comprising a T connection (13) that is able to match with any type of outer thread, allowing the simultaneous coupling of the vacuum pressure reader (12), vacuum pressure regulation valve (14) and aspiration line (15).

16. **"DEVICE TO CONTROL THE SUCTION PRESSURE IN THE SYSTEM PERFORMING ASPIRATION OF SECRETIONS FROM THE RESPIRATORY TRACT",** according to claim 13, comprising the aforesaid aspiration line that serves as a connector between the monitoring system elements, which possesses a smaller circuit (15), extending from the T connection (13) to the liquid separator container (17) and a larger circuit (16), extending from the liquid separator container (17) to the Y adaptor (19).

17. **"DEVICE TO CONTROL THE SUCTION PRESSURE IN THE SYSTEM PERFORMING ASPIRATION OF SECRETIONS FROM THE RESPIRATORY TRACT",** according to claim 13, comprising a liquid separator container (17), where the aspiration line circuits (15) and (16) are discharged.

18. **"DEVICE TO CONTROL THE SUCTION PRESSURE IN THE SYSTEM PERFORMING ASPIRATION OF SECRETIONS FROM THE RESPIRATORY TRACT",** according to claim 13, wherein the aforesaid liquid separator container (17) is designed to prevent that the sudden atmospheric air entry to push part of the fluid into the system flow toward the vacuum pressure reader.

19. **"DEVICE TO CONTROL THE SUCTION PRESSURE IN THE SYSTEM PERFORMING ASPIRATION OF SECRETIONS FROM THE RESPIRATORY TRACT",** according to claim 13, comprising an antimicrobial filter (18) placed in the aspiration line (15) circuit.

20. **"DEVICE TO CONTROL THE SUCTION PRESSURE IN THE SYSTEM PERFORMING ASPIRATION OF SECRETIONS FROM THE RESPIRATORY TRACT",** according to claim 13, wherein the aforesaid antimicrobial filter (18) is designed to prevent that solid or liquid residues may reach the pressure sensor/transducer, in this way reducing eventual malfunctioning or contamination by microorganisms.

21. **"DEVICE TO CONTROL THE SUCTION PRESSURE IN THE SYSTEM PERFORMING ASPIRATION OF SECRETIONS FROM THE RESPIRATORY TRACT",** according to claim 13, comprising a Y adaptor (19), connected to the extremity of the aspiration line circuit (16), which allows the coupling of the whole system together with the vacuum pressure generator and the aspiration catheter introduced into the patient's respiration route.

22. **"DEVICE TO CONTROL THE SUCTION PRESSURE IN THE SYSTEM PERFORMING ASPIRATION OF SECRETIONS FROM THE RESPIRATORY TRACT",** according to claim 13, wherein allows the vacuum pressure assessment during the secretion aspiration procedure so as to enable a real time analysis during such procedure.

23. **"DEVICE TO CONTROL THE SUCTION PRESSURE IN THE SYSTEM PERFORMING ASPIRATION OF SECRETIONS FROM THE RESPIRATORY TRACT",** according to claim 13, wherein allows the preventive inspections, maintenance and reassessments of the vacuum pressure regulating valve functioning.

24. **"DEVICE TO CONTROL THE SUCTION PRESSURE IN THE SYSTEM PERFORMING ASPIRATION OF SECRETIONS FROM THE RESPIRATORY TRACT",** according to claim 13, which is able to track the vacuum pressures (minimum, medium and maximum values) to be applied to patients.

25. **"DEVICE TO CONTROL THE SUCTION PRESSURE IN THE SYSTEM PERFORMING ASPIRATION OF SECRETIONS FROM THE RESPIRATORY TRACT",** according to claim 13, which is able to detect and quantify the pressure peaks in the system.

26. **"DEVICE TO CONTROL THE SUCTION PRESSURE IN THE SYSTEM PERFORMING ASPIRATION OF SECRETIONS FROM THE RESPIRATORY TRACT",** according to claim 13, which is able to track the vacuum pressure dynamic behavior all through the aspiration procedure.

27. **"DEVICE TO CONTROL THE SUCTION PRESSURE IN THE SYSTEM PERFORMING ASPIRATION OF SECRETIONS FROM THE RESPIRATORY TRACT",** according to claim 13, which is able to adjust the vacuum pressure under the aforesaid valve control.

28. **"DEVICE TO CONTROL THE SUCTION PRESSURE IN THE SYSTEM PERFORMING ASPIRATION OF SECRETIONS FROM THE RESPIRATORY TRACT", a**ccording to claim 13, which is able to quantify any charge loss in the antimicrobial filter, in this way showing the need for an eventual change of device components.

29. **"DEVICE TO CONTROL THE SUCTION PRESSURE IN THE SYSTEM PERFORMING ASPIRATION OF SECRETIONS FROM THE RESPIRATORY TRACT",** according to claim 13, wherein can be adapted to a large hospital systems, as well as to portable aspirators used in ambulatory and household environments.
